# DEMANDE DE BREVET EUROPEEN

(11) **EP 0 780 383 A1**
(43) Date de publication de la demande: **25.06.1997**
(21) Numéro de dépôt: 96402549.8
(22) Date de dépôt: 26.11.1996
(51) Int. Cl.: C07D 277/14, A61K 7/48

(54) **Nouveaux dérivés de l'acide L-2-oxothiazolidine 4-carboxylique et leur utilisation pour le soin de la peau**

(30) Priorité: 22.12.1995 FR 9515334
(71) Demandeur: L'OREAL, 75008 Paris (FR)
(72) Inventeur: Galey, Jean-Baptiste, 93600 Aulnay-sous-Bois (FR)
(74) Mandataire: Tezier Herman, Béatrice

(57) **Abrégé**

L'invention se rapporte à de nouveaux composés de formule (I) : 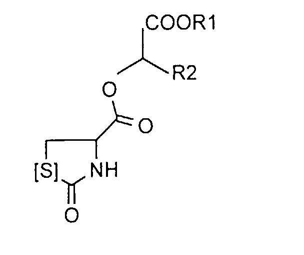
où R1 est choisi parmi l'hydrogène, les groupements alkyle en C1 à C8 linéaires ou ramifiés, saturés ou insaturés, éventuellement substitués par un substituant, les groupements benzyle éventuellement substitués, et
R2 est choisi parmi l'hydrogène, les groupements alkyle en C1 à C24, linéaires ou ramifiés, saturés ou insaturés éventuellement substitués par un substituant, les groupements aromatiques éventuellement substitués par un substituant, les groupements hétérocycliques, saturés ou insaturés.

Elle se rapporte également à une utilisation de ces dérivés pour le soin de la peau.

## Description

La présente invention se rapporte à de nouveaux dérivés de l'acide L-2-oxothiazolidine 4-carboxylique (encore appelé "procystéine") ainsi qu'à une composition les comprenant.

Elle se rapporte également à une utilisation de ces dérivés de l'acide L-2-oxothiazolidine 4-carboxylique comme précurseur de cystéine et d'hydroxyacide. Elle concerne aussi des utilisations de ces dérivés de l'acide L-2-oxothiazolidine 4-carboxylique pour le soin de la peau.

Les utilisateurs de produits cosmétiques et/ou dermatologiques cherchent de plus en plus à rajeunir, c'est-à-dire à diminuer les rides et les ridules, à illuminer le visage ainsi qu'à assouplir la peau. Ces signes du vieillissement sont non seulement dus à l'âge mais surtout dus à l'exposition solaire et à d'autres rayonnements tels que le bronzage artificiel par des U.V. notamment.

Parallèlement, ces mêmes utilisateurs cherchent de plus en plus à éclaircir leur peau.

Les actifs connus pour lutter contre le vieillissement sont l'acide rétinoïque et ses dérivés. Malheureusement, ces actifs présentent des effets secondaires comme des picotements, des échauffements et des rougeurs désagréables pour l'utilisateur et écartant les utilisateurs à peau sensible de ce type d'actif.

Il subsiste donc le besoin d'agents antivieillissement et/ou blanchissant à action aussi efficace que ceux de l'art antérieur mais ne présentant pas leurs inconvénients.

Par ailleurs, à ce jour il existe très peu d'actifs ayant des propriétés dépigmentantes.

La demanderesse a découvert de façon surprenante de nouveaux esters de l'acide L-2-oxothiazolidine 4-carboxylique permettant de prévenir et/ou lutter contre le vieillissement photoinduit ainsi que de dépigmenter ou blanchir la peau sans provoquer d'irritation de la peau. Les esters interviennent notamment au niveau de la synthèse de la mélanine ainsi que sur l'élimination des cellules mortes de la peau (ou desquamation).

Il est connu du document US 5 208 249 des esters de l'acide L-2-oxothiazolidine 4-carboxylique destinés à stimuler la synthèse intracellulaire du glutathion dans certaines cellules du corps humain. Ces esters comprennent un groupement ayant de 1 à 10 atomes de carbone et qui est choisi parmi les groupements méthyle, éthyle, propyle, isopropyle, butyle, isobutyle, tertiobutyle.

II est, par ailleurs, connu du document EP-A-656 201 d'autres esters de l'acide L-2-oxothiazolidine 4-carboxylique utilisés comme précurseur de cystéine et en association avec des stimulateurs de glutathion pour prévenir la chute des cheveux et stimuler la pousse de nouveaux cheveux. Ces esters encore différents des esters de ceux de l'invention ont un groupement alkyle, ayant de 1 à 10 atomes de carbone et choisi parmi les groupements méthyle, éthyle, propyle, butyle, pentyle, hexyle, peptyle, octyle, nonyle, décyle, isopropyle, isobutyle, sec-butyle, tert-butyle, isopentyle.

L'invention a donc pour objet un composé de formule générale (I) 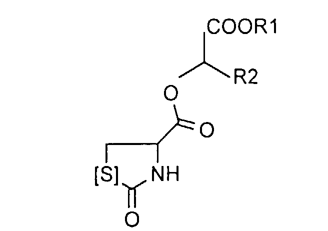 où R1 est choisi parmi l'hydrogène, les groupements alkyle en C1 à C8 linéaires ou ramifiés, saturés ou insaturés, éventuellement substitués par un substituant, les groupements benzyle éventuellement substitués, et

R2 est choisi parmi l'hydrogène, les groupements alkyle en C1 à C24, linéaires ou ramifiés, saturés ou insaturés éventuellement substitués par un substituant, les groupements aromatiques éventuellement substitués par un substituant, les groupements hétérocycliques.

Avantageusement, R1 est choisi parmi les groupements alkyle linéaires saturés ayant de 1 à 4 atomes de carbone et R2 est choisi parmi les groupements alkyle saturés ou insaturés ayant de 1 à 18 atomes de carbone et le groupements benzyle.

De préférence, R2 est choisi parmi les groupements alkyle saturés ou benzyle.

Le substituant dans R1 et R2 peut être choisi parmi les atomes de chlore, de fluor, les groupements OH, COOH, OR2, où R2 a la signification indiquée ci-dessus.

Le groupement hétérocyclique peut comporter un hétéroatome choisi parmi l'azote, l'oxygène, le soufre. Cet hétérocycle peut en outre être substitué par un des substituants ci-dessus.

De préférence, R1 est choisi parmi un groupement éthyle ou benzyle et R2 est choisi parmi un groupement méthyle, propyle, dodécyle, benzyle et l'hydrogène.

De façon préférée, le composé selon l'invention est choisi parmi l'ester 1-éthoxycarbonyl éthylique de l'acide 2-oxo-thiazolidine-4-carboxylique, l'ester 1-éthoxycarbonyl méthylique de l'acide 2-oxo-thiazolidine-4-carboxylique, l'ester 1-éthoxycarbonyl butylique de l'acide 2-oxo-thiazolidine-4-carboxylique, l'ester 1-éthoxycarbonyl phénylméthylique de l'acide 2-oxo-thiazolidine-4-carboxylique, l'ester 1-éthoxycarbonyl tridécylique de l'acide 2-oxo-thiazolidine-4-carboxylique, l'ester 1-benzyloxycarbonyl éthylique de l'acide 2-oxo-thiazolidine-4-carboxylique.

Un autre objet de l'invention est une composition cosmétique comprenant au moins un composé tel que défini précédemment.

L'invention a encore pour objet une utilisation comme précurseur de cystéine et/ou comme précurseur d'hydroxyacide, du composé de formule (I) susmentionné.

Un autre objet de l'invention est une utilisation d'un précurseur de cystéine et/ou d'hydroxyacide comme agent dépigmentant. Le précurseur peut être alors notamment le composé de formule (I).

Un des autres objets de l'invention est d'utiliser le composé de formule (I) susmentionné pour protéger le peau de l'irritation et/ou de la sensibilisation provoquée par des composants étrangers à l'organisme encore appelés "xénobiotiques".

L'invention a encore pout objet une utilisation d'un précurseur de cystéine et/ou d'hydroxyacide pour prévenir et/ou lutter contre le photovieillissement de la peau. Le précurseur peut être alors le composé de formule (I).

Les deux derniers objet de l'invention sont un procédé de traitement cosmétique qui consiste à appliquer sur la peau le composé de formule (I) dans un milieu cosmétiquement acceptable, et un procédé de fabrication de ce composé consistant à mélanger l'acide L-2-oxothiazolidine 4-carboxylique avec un ester α-bromé, en présence d'un équivalent molaire d'une base, puis à chauffer le mélange réactionnel, à le refroidir et à le filtrer.

Ce procédé de fabrication se caractérise de plus par une extraction des phases organiques, par leur séchage puis par leur purification. Cette base peut être choisie parmi la soude, la potasse, la méthanolamine.

Par milieu cosmétiquement acceptable, on comprend un milieu compatible avec la peau, les muqueuses, les ongles, les cheveux.

Le composé selon l'invention, une fois appliqué sur la peau, présente l'avantage d'apporter un hydroxyacide sous forme de précurseur, ainsi que de la cystéine également sous forme de précurseur stable, la cystéine entrant dans la constitution du glutathion.

En effet, le glutathion est composé des trois acides aminés suivants : acide glutamique, cystéine et glycine. Il est notamment connu pour intervenir dans la protection des cellules contre différents dommages tels que ceux dus aux radicaux libres (voir US 5 208 249). En outre, il permet l'inhibition de la production de mélanine mise en jeu dans la pigmentation de la peau.

Par ailleurs, le composé selon l'invention présente un caractère lipophile qui en facilite l'incorporation dans des compositions, notamment cosmétiques, pour former, par exemple, des émulsions de type huile-dans-eau ou eau-dans-huile.

Enfin, le composé selon l'invention possède d'excellentes qualités de pénétration dans la peau.

La composition selon l'invention peut, de plus, comprendre au moins un additif choisi parmi les corps gras, les conservateurs, les vitamines, les gélifiants, les parfums, les silicones, les tensioactifs, l'eau, les antioxydants, les charges, les filtres, les hydratants et leurs mélanges.

Les corps gras peuvent être choisis parmi les huiles telles que l'huile de jojoba, l'huile de vaseline, le palmitate d'isopropyl, ou parmi les cires telles que la cire de sipol, la cire de carnauba, les silicones, les acides gras, les alcools gras.

Le tensioactif éventuellement présent dans la composition peut être choisi parmi le mono et di-stéarate de glycéryl, le stéarate de sodium. Le gélifiant peut être choisi parmi les polyéthylène glycols éventuellement oxyéthylénés.

Le composé selon l'invention peut être présent en une quantité allant de 0,01 à 10 % en poids et de préférence en une quantité allant de 0,5 à 5 % en poids par rapport au poids total de la composition.

L'additif peut être présent dans la composition en une quantité allant de 0,01 à 5% en poids et de préférence en une quantité allant de 0,5 à 2 % en poids par rapport au poids total de la composition.

Le procédé de fabrication du composé (I) suit le schéma général suivant :

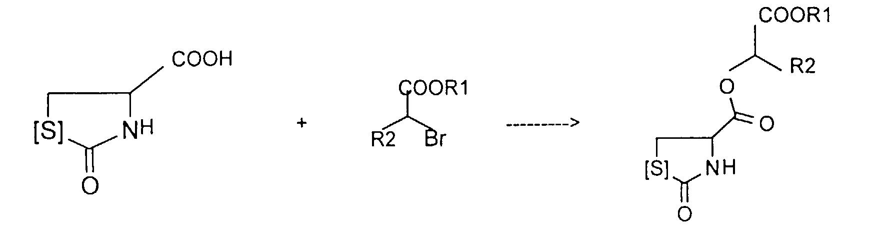

On donne ci-après le mode opératoire détaillé.

Dans un réacteur de 100 ml, on, introduit 20 millimoles d'acide L-2-oxothiazolidine 4-carboxylique dans 30 ml de diméthylformamide anhydre sous agitation. On ajoute 10 mmoles de K₂CO₃ et on chauffe le mélange pendant 20 minutes à 90°C. On additionne alors 22 mmoles d'ester α-bromé. Le mélange est chauffé à 80°C de une heure à 4 heures selon les cas et ceci jusqu'à disparition complète des produits de départ en suivant par chromatographie en couche mince. Le mélange réactionnel est alors refroidi, puis filtré pour éliminer les sels.

Le filtrat est versé dans 120 ml d'eau saturée de NaCI et extrait par 3 fois par 100 ml de dichlorométhane. Les phases organiques sont rassemblées puis lavées à l'eau. La phase organique est enfin séchée sur du sulfate de sodium puis évaporée à sec. L'huile obtenue est purifiée par chromatographie sur colonne de silice en éluant au dichlorométhane.

On obtient différents composés selon la nature de l'ester bromé utilisé.

### Composé 1: Ester 1-éthoxycarbonyl éthylique de l'acide 2-oxo-thiazolidine-4-carboxylique - R1 = éthyle et R2 = méthyle

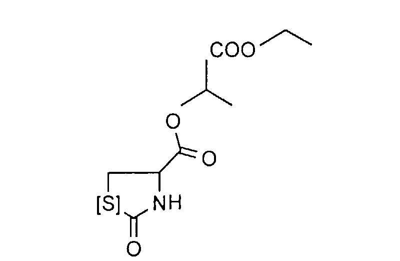

Le produit se présente sous forme huileuse et a un spectre RMN ¹H conforme à la structure attendue.

| Analyse élémentaire : | | | | | |
|---|---|---|---|---|---|
| | C | H | N | O | S |
| Théorique | 43,72 | 5,30 | 5,66 | 32,35 | 12,97 |
| Calculée | 43,92 | 5,40 | 5,62 | 32,66 | 13,01 |

### Composé 2 : Ester 1-éthoxycarbonyl méthylique de l'acide 2-oxo-thiazolidine-4-carboxylique - R1 = éthyle et R2 = H

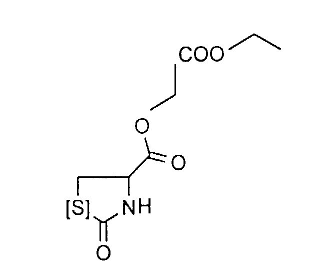

Le produit se présente sous forme huileuse et a un spectre RMN ¹H conforme à la structure attendue.

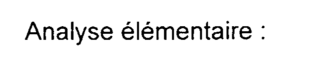

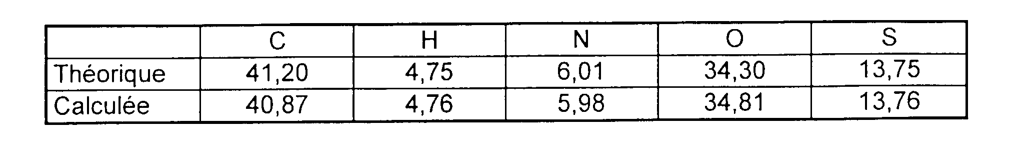

### Composé 3 : Ester 1-éthoxycarbonyl butylique de l'acide 2-oxo-thiazolidine-4-carboxylique - R1 = éthyle - R2 = propyle

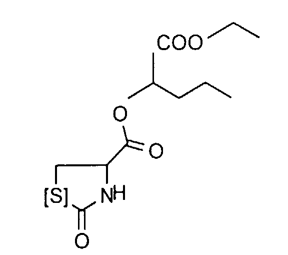

Le produit se présente sous forme huileuse et a un spectre RMN ¹H conforme à la structure attendue.

| Analyse élémentaire : | | | | | |
|---|---|---|---|---|---|
| | C | H | N | O | S |
| Théorique | 47,99 | 6,22 | 5,09 | 29,06 | 11,65 |
| Calculée | 47,90 | 6,27 | 5,12 | 29,21 | 11,40 |

### Composé 4 : Ester 1-éthoxycarbonyl phényl méthylique de l'acide 2-oxo-thiazolidine-4-carboxylique - R1 = éthyle et R2 = phényle

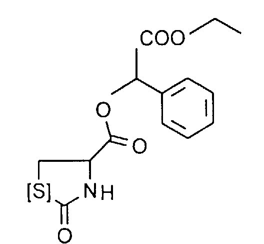

Le produit se présente sous forme de cire. Le spectre RMN ¹H est conforme à la structure attendue.

| Analyse élémentaire : | | | | | |
|---|---|---|---|---|---|
| | C | H | N | O | S |
| Théorique | 54,36 | 4,89 | 4,53 | 25,86 | 10,37 |
| Calculée | 54,40 | 4,89 | 4,51 | 26,05 | 10,31 |

### Composé 5 : Ester 1-éthoxycarbonyl tridécylique de l'acide 2-oxo-thiazolidine-4-carboxylique - R1 = éthyle et R2 = dodécyle

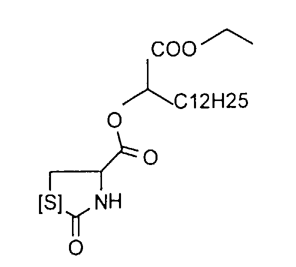

Le produit se présente sous forme huileuse et a un spectre RMN ¹H conforme à la structure attendue.

| Analyse élémentaire : | | | | | |
|---|---|---|---|---|---|
| | C | H | N | O | S |
| Théorique | 59,82 | 8,79 | 3,49 | 19,92 | 7,98 |
| Calculée | 59,79 | 8,75 | 3,50 | 20,01 | 8,07 |

### Composé 6 : Ester 1-benzyloxycarbonyl éthylique de l'acide 2-oxo-thiazolidine-4-carboxylique - R1 = benzyle et R2 = méthyle

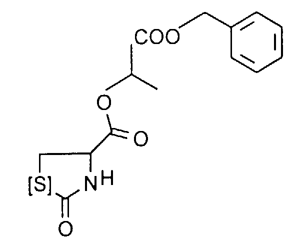

Le produit se présente sous forme huileuse et a un spectre RMN ¹H conforme à la structure attendue.

| Analyse élémentaire : | | | | | |
|---|---|---|---|---|---|
| | C | H | N | O | S |
| Théorique | 54,36 | 4,89 | 4,53 | 25,86 | 10,37 |

L'invention est illustrée plus en détail à l'aide des exemples qui suivent. Ces différentes compositions sont préparées selon les techniques classiques.

Les concentrations sont données en pourcentage en poids par rapport au poids total de la composition.

### Exemple 1 : Crème de soin protectrice

- Ester 1-éthoxycarbonyl éthylique de l'acide 2-oxo-thiazolidine-4-carboxylique (composé 1) 1
- Polyéthylène glycol 50 oxyéthyléné 3
- Mono diglycéryl stéarate 3
- Huile de vaseline 24
- Acool cétylique 5
- Eau qsp 100

### Exemple 2 : Crème de soin pour le corps

- Ester 1-éthoxycarbonyl éthylique de l'acide 2-oxo-thiazolidine-4-carboxylique (composé 1) 0,5
- Cire de sipol 6
- Glycéryl monostéarate 1,5
- Stéarate de sodium 0,8
- Huile de vaseline 6
- Palmitate d'isopropyle 2
- Glycérol 15
- Parfum 0,3
- Eau qsp 100

### Exemple 3 :Crème de soin blanchissante

- Ester 1-éthoxycarbonyl éthylique de l'acide 2-oxo-thiazolidine-4-carboxylique (composé 1) 0,5
- Huile de jojoba 13
- Alkyl paraben 0,05
- Sorbate de potassium 0,3
- Cyclopenta diméthylsiloxane 10
- Alcool stéarylique 1
- Acide stéarique 4
- Stéarate de polyéthylène glycol 3
- Vitamine E 1
- Glycérol 3
- Eau qsp 100

## Revendications

1. Composé de formule générale (I) 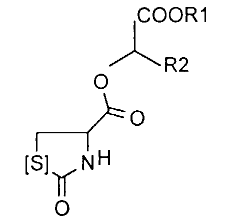 où
R1 est choisi parmi l'hydrogène, les groupements alkyle en C1 à C8 linéaires ou ramifiés, saturés ou insaturés, éventuellement substitués par un substituant, les groupements benzyle éventuellement substitués, et
R2 est choisi parmi l'hydrogène, les groupements alkyle en C1 à C24, linéaires ou ramifiés, saturés ou insaturés éventuellement substitués par un substituant, les groupements aromatiques éventuellement substitués par un substituant, les groupements hétérocycliques, saturés ou insaturés.

2. Composé selon la revendication 1, caractérisé en ce que R1 est choisi parmi les groupements alkyle linéaires saturés ayant de 1 à 4 atomes de carbone.

3. Composé selon la revendication 1 ou 2, caractérisé en ce que R2 est choisi parmi les groupements alkyle saturés ou insaturés ayant de 1 à 18 atomes de carbone, et le groupement benzyle.

4. Composé selon l'une des revendications 1 ou 2, caractérisé en ce que R2 est choisi parmi les groupements alkyle saturés ou benzyle.

5. Composé selon la revendication 1, caractérisé en ce que le substituant est choisi parmi, les atomes de chlore, de fluor, les groupements OH, COOH et OR2.

6. Composé selon la revendication 1, caractérisé en ce que le groupement hétérocyclique comporte un hétéroatome choisi parmi l'azote, l'oxygène, le soufre.

7. Composé selon l'une des revendications précédentes, caractérisé en ce que R1 est choisi parmi les groupements éthyle et benzyle.

8. Composé selon l'une des revendications précédentes, caractérisé en ce que R2 est choisi parmi les groupements méthyle, propyle, dodécyle, benzyle et l'hydrogène.

9. Composé selon l'une des revendications précédentes, caractérisé en ce qu'il est choisi parmi l'ester 1-éthoxycarbonyl éthylique de l'acide 2-oxo-thiazolidine-4-carboxylique, l'ester 1-éthoxycarbonyl méthylique de l'acide 2-oxo-thiazolidine-4-carboxylique, l'ester 1-éthoxycarbonyl butylique de l'acide 2-oxo-thiazolidine-4-carboxylique, l'ester 1-éthoxycarbonyl phénylméthylique de l'acide 2-oxo-thiazolidine-4-carboxylique, l'ester 1-éthoxycarbonyl tridécylique de l'acide 2-oxo-thiazolidine-4-carboxylique, l'ester 1-benzyloxycarbonyl éthylique de l'acide 2-oxo-thiazolidine-4-carboxylique.

10. Composition caractérisée en ce qu'elle comprend au moins un composé selon l'une des revendications précédentes.

11. Composition selon la revendication 10, caractérisée en ce qu'elle comprend de plus au moins un additif choisi parmi les corps gras, les conservateurs, les vitamines, les gélifiants, les parfums, les silicones, les tensioactifs, l'eau, les antioxydants, les charges, les filtres.

12. Composition selon la revendication 11, caractérisée en ce que le corps gras est choisi parmi les huiles, les cires.

13. Composition selon la revendication 11, caractérisée en ce que le tensioactif est choisi parmi le mono et di-stéarate de glycéryl, le stéarate de sodium.

14. Composition selon la revendication 11, caractérisée en ce que le gélifiant est choisi parmi les polyéthylène glycols.

15. Composition selon l'une des revendications 10 à 14, caractérisée en ce que le composé est présent en une quantité allant de 0,01 à 10 % en poids par rapport au poids total de la composition.

16. Composition selon la revendication 15, caractérisée en ce que le composé est présent en une quantité allant de 0,5 à 5 % en poids par rapport au poids total de la composition.

17. Composition selon l'une des revendications 11 à 16, caractérisée en ce que l'additif est présent en une quantité allant de 0,01 à 5 % en poids par rapport au poids total de la composition.

18. Utilisation comme précurseur de cystéine et/ou comme précurseur d'hydroxyacide, du composé selon l'une quelconque des revendications 1 à 9.

19. Utilisation d'un précurseur de cystéine et/ou d'hydroxyacide comme agent dépigmentant.

20. Utilisation du composé selon l'une des revendications 1 à 9 pour protéger la peau de l'irritation et/ou de la sensibilisation provoquée par des composants étrangers à l'organisme.

21. Utilisation d'un précurseur de cystéine et/ou d'hydroxyacide pour prévenir et/ou lutter contre le photovieillissement de la peau.

22. Utilisation selon la revendication 19 ou 21, caractérisée en ce que le précurseur est le composé selon l'une des revendications 1 à 9.

23. Procédé de traitement cosmétique, caractérisé en ce qu'il consiste à appliquer sur la peau le composé de formule (I) dans un milieu cosmétiquement acceptable.

24. Procédé de fabrication du composé de formule (I), caractérisé en ce qu'il consiste à :
- mélanger l'acide L-2-oxothiazolidine 4-carboxylique avec un ester α-bromé, en présence d'un équivalent molaire d'une base ;
- chauffer le mélange réactionnel, à le refroidir et le filtrer.

25. Procédé selon la revendication 24, caractérisé en ce qu'il consiste à extraire les phases organiques, à les sécher et à les purifier.
